# EUROPÄISCHE PATENTSCHRIFT

(11) **EP 0 975 949 B1**
(45) Veröffentlichungstag und Bekanntmachung des Hinweises auf die Patenterteilung: **25.07.2001**
(21) Anmeldenummer: 98928163.9
(22) Anmeldetag: 07.05.1998
(51) Int. Cl.: G01N 13/00, G01N 33/15

(54) **VORRICHTUNG ZUM BESTIMMEN DER ZERFALLSZEIT VON VERPRESSTEN ARZNEIFORMKÖRPERN, WIE TABLETTEN UND KAPSELN, SOWIE VERFAHREN HIERZU**
DEVICE FOR DETERMINING THE DECAY TIME OF COMPRESSED MEDICAMENTS SUCH AS TABLETS AND CAPSULES, AND CORRESPONDING METHOD
DISPOSITIF POUR DETERMINER LE TEMPS DE DESAGREGATION DE CORPS MOULES MEDICAMENTEUX COMPACTES TELS QUE DES COMPRIMES ET DES CAPSULES, ET PROCEDE CORRESPONDANT

(30) Priorität: 07.05.1997 DE 19719201
(43) Veröffentlichungstag der Anmeldung: 02.02.2000
(73) Patentinhaber: Krämer, Norbert, 64291 Darmstadt (DE)
(72) Erfinder: Krämer, Norbert, 64291 Darmstadt (DE)
(74) Vertreter: Mierswa, Klaus, Dipl.-Ing.
(86) Internationale Anmeldenummer: DE9801282
(87) Internationale Veröffentlichungsnummer: WO9850776

(56) Entgegenhaltungen:
- WO-A-97/14035
- CH-A- 536 494
- DE-A- 3 414 507
- DE-C- 3 520 034
- DE-U- 9 419 245
- US-A- 3 618 395

## Beschreibung

Die Erfindung betrifft eine Vorrichtung zum Bestimmen der Zerfallszeit von verpreßten Arzneiformkörpern, wie Tabletten und Kapseln, bestehend aus einem in ein mit einer Flüssigkeit gefülltes Gefäß absenkbares Gestell, mit einer Mittelsäule und einem Boden, der eine Mehrzahl von Löchern enthält, die von unten mit einem Sieb abgedeckt sind und in denen innerhalb des Gestells Prüfröhrchen aufrechtstehend angeordnet sind, in welchen je eine Scheibe mit durchgehenden, in Richtung der Mittelachse der Scheibe verlaufende Löcher als Beschwerung des einzelnen Arzneiformkörpers beweglich einbringbar ist, wobei an der dem Boden zugewandten Unterseite der Scheibe ein Dauermagnet angeordnet ist, demgegenüber sich unterhalb des Netzes des Bodens je ein Magnetfeldsensorelement befindet, gemäß dem Oberbegriff des Anspruchs 1 sowie ein Verfahren hierzu.

### Stand der Technik:

Die Zerfallszeitmessung von verpreßten Arzneiformkörpern, wie Tabletten und Kapseln, erfolgt in einem standardisierten Versuchsaufbau, um die Reproduzierbarkeit der Meßergebnisse zu gewährleisten, insbesondere nach DAB 10, 3. Nachtrag 1994 oder Europäisches Arzneibuch oder USP (USA). Durch die Zerfallsprüfung wird festgestellt, ob die Tabletten oder Kapseln in der vorgeschriebenen Zeit unter genau ausgeführten Bedingungen in einem flüssigen Medium zerfallen. Der Hauptteil der Apparatur besteht aus einem starren Gestell, welches sechs zylindrische Prüfröhrchen aus Glas enthält. Jedes Röhrchen ist mit einer zylindrischen Scheibe aus durchsichtigem Kunststoffmaterial genau vorgeschriebener relativer Dichte und Größe versehen, welche 5 durchgehende Bohrungen aufweist, von denen eine Bohrung durch die Mittelachse der Scheibe geführt ist. Die Prüfröhrchen werden durch eine obere und eine untere durchsichtige Platte aus Kunststoffmaterial senkrecht gehaltert, die je sechs Bohrungen haben. Alle Bohrungen haben den gleichen Abstand vom Mittelpunkt und gleichen Abstand voneinander. Auf der Unterseite der unteren Platte befindet sich ein Sieb aus rostfreiem Stahldraht. In der Mitte der Platten ist eine Metallsäule so angebracht, daß die Apparatur an dieser Metallsäule in einer Aufhängevorrichtung aufgehängt und mittels eines Motors gleichmäßig 28 bis 32 mal je Minute 50 bis 60 mm hoch auf- und abbewegt werden kann. Die Apparatur wird in einem geeigneten Gefäß aufgehängt, welches die vorgeschriebene Flüssigkeit enthält. Nach dem Einfüllen einer Tablette oder Kapsel in jedes Röhrchen und Auflegen der Scheibe als Beschwerung erfolgt die Bestimmung der Auflösezeit der Tabletten oder Kapseln durch Beobachtung der Meßvorrichtung und Zeitnahme der Zerfallszeit durch die Bedienungsperson.

Durch die DE-C-35 20 034 ist ein Zerfallsgerät für Prüfkörper, insbesondere Tabletten, bekannt geworden, bei dem die Prüfkörper in Behältern eines Zerfallskorbes zwischen einem Hallgenerator und einer mit einem Magneten versehenen Scheibe angeordnet sind. Die Behälter des Zerfallskorbes werden mittels einer Heizung auf eine konstante Temperatur aufgeheizt. Wenn der Prüfkörper zerfällt, bewegt sich die Scheibe gemeinsam mit dem Magneten auf den Hallgenerator zu, so daß dieser ein Signal abgibt, welches nach dem Überschreiten einer Schaltschwelle einem Registriergerät zugeführt und angezeigt werden kann. Die Übertragung der Energie für elektrische Schaltungen im Zerfallskorb erfolgt über Kontakte oder durch einen Hochfrequenzsender und einen Hochfrequenzempfänger. Die Übertragung der Signale zum Registriergerät erfolgt durch optoelektrische Bauteile.

Durch die DE 94 19 245 U1 ist ein automatisches Zerfallszeit-Meßgerät für die pharmazeutische Qualitäts- und Produktionskontrolle von Tabletten und Dragees innerhalb einer leitfähigen Testflüssigkeit bekannt, welches aus einem in einem Becherglas angeordneten korbartigen Gestell besteht mit einer darin angeordneten Anzahl von Glasröhren, deren Böden durch kreisförmige Siebplatten als Standflächen für die Glasröhren gebildet werden, wobei jede Siebplatte aus zwei stromdurchflossenen elektrodenbildenden Drahtgeflechtshälften besteht, die unter Ausbildung eines Schlitzes in Abstand voneinander angeordnet sind. In jeder Glasröhre wird ein Prüfling angeordnet, der mittels eines Schwimmers abgedeckt ist, der auf dem Prüfling aufliegt. Der Schwimmer weist auf der Unterseite ein eingebettetes Kontaktgerüst aus einem metallischen Werkstoff auf. Bei Bewegung des Becherglases und Zerfall der Tabletten ändert sich die Leitfähigkeit der Testflüssigkeit, die zwischen den Drahtgeflechtshälften und dem Kontaktgerüst des Schwimmers gemessen werden kann.

Durch die US 3,618,395 ist ein Tabletten-Zerfallszeitmeßgerät mit bewegten Röhrchen in einer Badflüssigkeit bekannt mit einer Vielzahl von sich gegenüberliegenden beabstandeten Elektroden auf dem Boden der die Tabletten enthaltenden Röhrchen. Die Gegenwart einer Tablette stört ein an die Elektroden angelegtes elektromagnetisches Feld, wobei die Störung einen Zeitgeber beeinflußt, dessen Signale ausgewertet werden können.

Aus der WO 97/140 35 ist eine Vorrichtung zum Bestimmen der Zerfallszeit von verpreßten Arzneiformkörpern bekannt, bei der auf dem Boden des Gestells um jedes Loch eine elektrische Spule angeordnet ist, die Teil eines elektrischen Schwingkreises ist, wobei auf der Scheibe eine Leiterschleife zur wegabhängigen Dämpfung des elektrischen Schwingkreises angeordnet ist, die gemeinsam an eine elektrische Auswerteeinrichtung zur Schwingungserzeugung und Auswertung der Meßergebnisse angeschlossen sind. Die Spule kann als Einlagen- oder Mehrlagenspule ausgebildet sein.

Bisherige Ansätze für eine vollautomatische Messung gingen einerseits immer mit nicht zulässigen Änderungen der Versuchsapparatur einher, wobei derartige Änderungen jedoch gemäß DAB 10 nur in sehr geringem Umfang zulässig sind. Andererseits liefert die Vorrichtung gemäß der letztgenannten WO 97/140 35 relativ genaue Meßergebnisse, erfordert jedoch einen erheblichen elektronischen Aufwand in der Signalerzeugung und - auswertung.

### Technische Aufgabe:

Der Erfindung liegt die Aufgabe zugrunde, eine Vorrichtung der eingangs genannten Gattung zu schaffen, die ohne größere Änderungen der vorgeschriebenen Parameter für die Apparatur gemäß DAB 10 berührungslos die Bewegung der Scheiben innerhalb der Vorrichtung erfassen und kontinuierlich die beim Auflösungsvorgang abnehmende Dicke von verpreßten Arzneiformkörper ermitteln soll.

Die Lösung der Aufgabe besteht erfindungsgemäß darin, dass das zentrale Loch der Scheibe zentrisch eine von der unteren Deckfläche der Scheibe ausgehende Aufbohrung aufweist, in der der Dauermagnet sitzt, der zentrisch in Richtung der Mittelachse ein Loch aufweist, welches mit dem zentralen Loch innerhalb der Scheibe fluchtet, wobei das Magnetfeldsensorelement in der Draufsicht auf den Dauermagneten im Bereich des peripheren Randes der Deckfläche des Dauermagneten angeordnet ist. Vorteilhaft ist das Magnetfeldsensorelement im Bereich des größten Gradienten des magnetischen Feldes des Dauermagneten angeordnet.

Normalerweise wird die Restdicke eines Arzneiformkörpers als Sollwert vorgegeben. Wenn der Istwert des Arzneiformkörpers als die Restdicke ist, so gilt derselbe als aufgelöst.

Vorteilhaft ist die Vorrichtung so gestaltet, daß die Geometrie und die Dichte der verwendeten Scheiben sowie die Geometrie der Prüfröhrchen und der Aufbau des Siebbodens des Gestells unverändert ist. Ebenso sind die Scheiben und die Gestelle untereinander austauschbar, ohne daß eine Neukalibrierung der Vorrichtung durchgeführt werden muß. Insbesondere können solche Scheiben verwendet werden, die vorgeschriebenermaßen auch fünf Löcher aufweisen, von denen eines ein zentrales Mittelloch ist. Nur sind die Scheiben derart gestaltet, daß um die zentrale Mittelbohrung innerhalb der Scheibe herum sich ein Dauermagnet mit einem Loch befindet, so daß sich das zentrale Loch der Scheibe durch das Loch des Dauermagneten fortsetzt. Die geringfügige Gewichtszunahme durch den Dauermagneten kann in einfacher Weise durch die Einbringung kleiner Hohlräume in die Scheibe ausgeglichen werden, so daß das vorgeschriebene Gewicht der Scheibe eingehalten wird.

Vorzugsweise ist der Dauermagnet rohr- oder hülsenförmig zylindrisch gestaltet, wobei seine Längenausdehnung größer als sein Durchmesser ist. Auf diese Weise kann die magnetische Streuung des Dauermagneten gering gehalten werden. Innerhalb der Scheibe ist der Dauermagnet vollständig von Kunststoffmaterial umgeben, zum Beispiel ist in das Loch des Dauermagneten eine Kunststoffhülse eingesetzt.

Das Magnetfeldsensorelement kann ein Hallgenerator oder ein magnetoresistiver Sensor oder eine Sättigungskernsonde sein, bevorzugt ist es ein Hallgenerator, der schon geringfügigste Feldveränderungen zu messen imstande ist.

Das Magnetfeldsensorelement bzw. der Hallgenerator ist im Bereich des größten Gradienten des Magnetfeldes angeordnet, sobald der Dauermagnet in der Endstellung der Scheibe dem Magnetfeldsensorelement direkt gegenüber steht. Aus diesem Grund ist es vorteilhaft, das Magnetfeldsensorelement räumlich so weit zu versetzen, daß der Hauptteil des Sensors mit kleinen Abmessungen direkt unterhalb der ringförmigen Stirnfläche des Magneten sich befindet, also in Draufsicht seitlich des Loches des Dauermagneten.

Desweiteren wird mit der Vorrichtung jeder Kanal während eines Zerfallsvorgangs mehrfach gemessen und daraus ein Mittelwert gebildet. Es ist auch möglich, jedes der sechs Magnetfeldsensorelemente mittels eines schnellen Multiplexers abzufragen.

Kurzbeschreibung der Zeichnung, in der zeigen:
- Figur 1 a, b, c: eine Vorrichtung des Standes der Technik gem. DAB 10
- Figur 2: eine Scheibe in Draufsicht, wie sie in jedes Prüfröhrchen eingesetzt wird, mit einem hülsenförmigen Magneten
- Figur 3: eine Seitenansicht der Figur 2 mit der Anordnung des Hallgenerators sowie der nachgeschalteten elektronischen Auswerteschaltung für die Meßsignale
- Figur 4: eine Seitenansicht einer erfindungsgemäßen Vorrichtung, wobei von den Prüfröhrchen nur eines gezeigt ist und
- Figur 5: eine vergrößerte Darstellung des Dauermagneten und der Anordnung des magnetischen Sensors am peripheren Rand der unteren Stirnfläche des Dauermagneten.

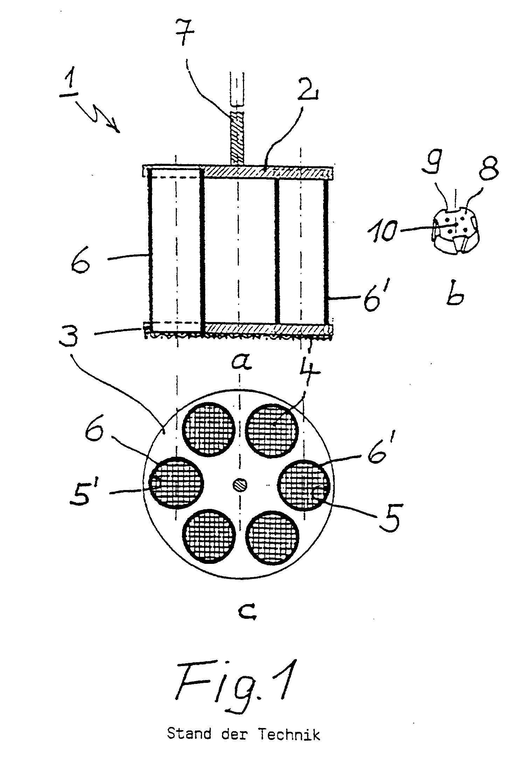

Die Figur 1 a, b und c zeigt eine Vorrichtung gemäß DAB 10, 3. Nachtrag 1994, wie sie in der vorliegenden Erfindung verwendet wird. Die Vorrichtung besteht aus einem Gestell 1 mit einer Mittelsäule 7 sowie einer kreisrunden Abdeckplatte 2 und einem Boden 3, die eine Mehrzahl von Löchern 5, 5', in der Regel sechs, aufweisen, in denen Prüfröhrchen 6, 6' aus Glas angeordnet sind. Der Boden 3 ist auf der Unterseite mit einem Sieb 4 aus rostfreiem Stahldraht abgedeckt. Die Platten 2, 3 sind voneinander durch nicht gezeigte senkrechte Metallstäbe an der Außenseite des Gestells 1 starr gehalten. Zur Zerfallsprüfung wird jeweils eine Tablette oder Kapsel in jedes Prüfröhrchen 6, 6' gelegt und eine Scheibe, die genau definierte Aussparungen 9 sowie vier bis fünf Bohrungen 10, 10' besitzt, auf die Tablette oder Kapsel als Beschwerung aufgelegt und die Zerfallszeit gemessen. Vorzugsweise besitzt die Scheibe 8 eine Mittelbohrung 10, die längs der Mittelachse der Scheibe 8 verläuft.

### Bevorzugte Ausführung der Erfindung:

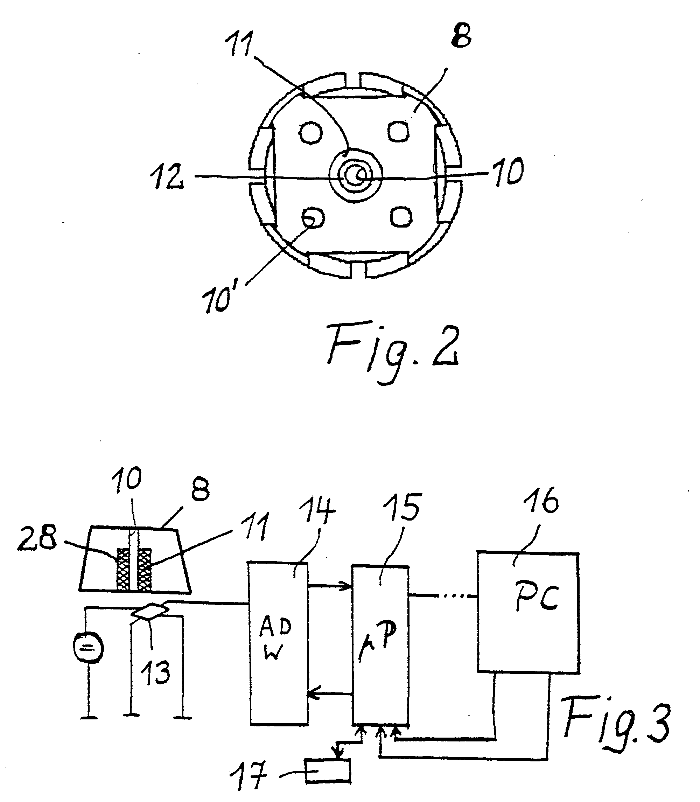

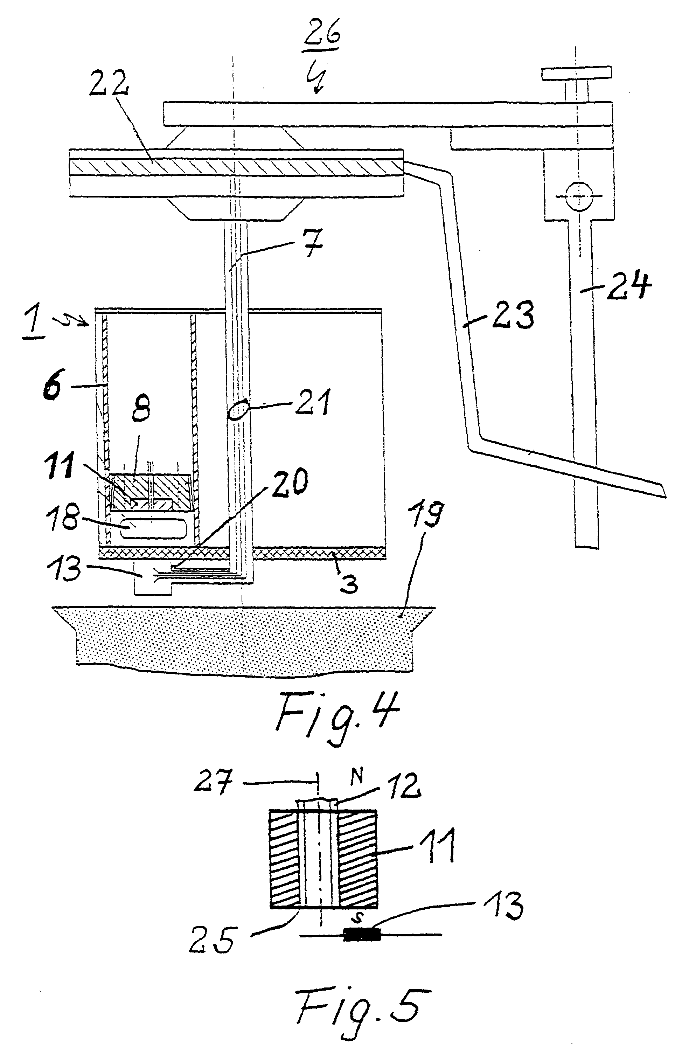

Figur 2 zeigt eine erfindungsgemäße Scheibe, die ähnlich der Scheibe in Figur 1 b gestaltet ist. Der Unterschied zu dieser Scheibe besteht darin, daß die Scheibe 8 der Figur 2 längs der Mittelachse einen Dauermagneten 11 aufweist, der ein Loch 25 (Figur 5) besitzt und somit als Torus ringförmig gestaltet ist, welches sich ebenfalls in Richtung der Mittelachse der Scheibe 8 bzw. in Richtung der Mittelachse 27 (Figur 5) des Dauermagneten 11 erstreckt. Der Dauermagnet 11 ist vollständig in das Material der Scheibe 8 eingebettet und von diesem umgeben und zwar vorzugsweise von der größeren Unterseite der Scheibe 8 ausgehend, wie es der Figur 3 zu entnehmen ist. Dazu weist das zentrale Loch 10 der Scheibe 8 zentrisch eine von der unteren Deckfläche der Scheibe 8 ausgehende Aufbohrung 28 auf, in der der Dauermagnet 11 sitzt, der zentrisch in Richtung der Mittelachse 27 das Loch 25 aufweist, welches mit dem zentralen Loch 10 innerhalb der Scheibe 8 fluchtet, wobei das Magnetfeldsensorelement 13 in der Draufsicht auf den Dauermagneten 11 im Bereich des peripheren Randes der Deckfläche des Dauermagneten 11 im Bereich des größten Gradienten des magnetischen Feldes des Dauermagneten 11 angeordnet ist.

In das Loch 25 des Dauermagneten 11, welches eine Bohrung sein kann, ist ein Kunststoffröhrchen 12 eingesetzt, welches den Dauermagneten in dessen Länge überragt und bis zur Oberkante der oberen Oberfläche der Scheibe 8 reicht. Die untere Stirnfläche des Dauermagneten 11 ist ebenfalls mit einem dünnen Kunststoffüberzug abgedeckt, so daß auf diese Weise der Dauermagnet 11 vollständig von Kunststoff umschlossen innerhalb der Scheibe 8 eingebettet angeordnet ist.

Gemäß den Figuren 3 und 5 ist der Dauermagnet 11 vorzugsweise röhren- oder hülsenförmig zylindrisch gestaltet, wobei seine Längenausdehnung größer als der Durchmesser ist. Auf diese Weise wird erreicht, daß der Dauermagnet 11 an seinen Polen N-S ein Magnetfeld mit einem starken Gradienten in den unmittelbaren Bereichen der Stirnflächen ausbildet.

Das Magnetfeldsensorelement ist in der Lage, auch relativ schwache Magnetfelder ausreichend zu erfassen, insbesondere wenn das Magnetfeldsensorelement im Bereich des größten Gradienten des Magnetfeldes angeordnet ist, sobald die Scheibe 8 mitsamt dem Dauermagneten 11 die untere Endstellung innerhalb des Prüfröhrchens 6 erreicht hat. Als Magnetfeldsensorelement kann ein Hallgenerator oder ein magnetoresistiver Sensor oder eine Sättigungskernsonde verwendet werden. Sättigungskernsonden sind Positionssensoren aus einem speziellen, sehr kleinen weichmagnetischen Kern, der von einer Spule umwickelt ist. Diese Spule erlaubt die Bestimmung des Magnetisierungszustandes des Sensorkerns durch den Dauermagneten mit Hilfe einer externen Ansteuerungs- und Auswerteelektronik.

Das verwendete Magnetfeldsensorelement muß eine hohe Magnetfeldempfindlichkeit sowie eine geringe Bauhöhe wie auch eine Ansteuerung durch den Dauermagneten aufweisen, die sehr flexibel an die Anwendung angepaßt werden kann. Ebenso ist es empfehlenswert, für den Dauermagneten 11 ein Material zu verwenden, welches eine hohe magnetische Permeabilität besitzt. Als Magnetmaterial eignet sich beispielsweise N-30 H.

Figur 3 zeigt desweiteren den elektrischen Anschluß des Magnetfeldsensorelements 13, welches hier als Hallgenerator 13 mit einer Versorgungsspannung dargestellt ist. Der Hallgenerator 13 liefert an seinem Ausgang eine Spannung, die der Stärke des Magnetfeldes des Dauermagneten 11 sowie dem Abstand desselben von dem Hallgenerator 13 entspricht.

Ebenso kann als Magnetfeldsensorelement ein magnetischer Widerstand verwendet werden, wobei hier das Ausgangssignal als ein Spannungsabfall am magnetischen Widerstand gewonnen wird. Das jeweilige Ausgangssignal des Hallgenerators 13 bzw. des magnetischen Widerstandes werden einem AD-Wandler 14 und von dort einem Mikroprozessor 15 zugeführt und digitalisiert. Der Mikroprozessor 15 ist mit einem Rechner 16 verbunden, über den der Zerfallsvorgang angezeigt und überwacht werden kann.

Zur Erhöhung der Empfindlichkeit der Messung kann der Mikroprozessor 15 den AD-Wandler 14 veranlassen, mehrmals den entsprechenden Kanal zu messen. Ein Speicherelement RAM 17, welches vorzugsweise gepuffert ist, ist für die dauerhafte Speicherung der Endwerte und der sich ändernden Dicke des sich auflösenden Arzneiformkörpers vorgesehen. Die Zerfallszeit wird mit dem angeschlossenen Rechner 16 festgestellt.

Figur 4 zeigt eine Seitenansicht einer Vorrichtung, wobei von dem Prüfröhrchen 6 nur eines gezeigt ist. Das absenkbare Gestell 1 ist über einem Gefäß 19 angeordnet, in welchem sich eine Flüssigkeit befindet, vorzugsweise Wasser. Innerhalb des Prüfröhrchen 6 befindet sich ein Arzneiformkörper 18 sowie darüber eine Scheibe 8, welche einen Dauermagneten 11 aufweist. Unterhalb des Bodens 3 befindet sich das Magnetfeldsensorelement 13, dessen elektrische Zuleitungen 21 durch die Mittelsäule 7 des Gestells 1 geführt sind und mit einer Platine 22 verbunden sind, welche Teil eines Kopfteils 26 der gesamten Apparatur ist. Das Kopfteil 26 ist an einer senkrechten Säule 24 befestigt, die das Absenken und Anheben des Kopfteils 26 mitsamt dem Gestell 1 in das Gefäß 19 ermöglicht. Ein Kabel 23 führt zum Rechner 16, der in Figur 3 gezeigt ist.

Desweiteren ist in Figur 4 im Bereich des Magnetfeldsensorelementes 13 ein Temperatursensor 20 angeordnet, zur Erfassung und Steuerung der Temperatur der Flüssigkeit innerhalb des Gefäßes 19.

Das Meßverfahren ist dergestalt, daß während des Auflösungsvorgangs des Arzneiformkörpers eine elektrische Mehrfachmessung mit anschließender Mittelwertbildung erfolgt. Dabei wird das sich ändernde Magnetfeld des Dauermagneten 11 in Abhängigkeit des momentanen Abstandes desselben vom Magnetfeldsensorelement 13 sowie von der Zerfalls- oder Sinkzeit oder nur von der Zerfallszeit gemessen. Der momentane Wert des Magnetfeldes bei sich auflösenden Arzneiformkörper ist ein Maß über die momentane Höhe des Dauermagneten 11 über dem Magnetfeldsensorelement 13 und somit über die momentane Restdicke des Arzneiformkörpers 18. Die Erfassung des maximalen Magnetfeldes ist gleichzusetzen mit der vollständigen Auflösung Arzneiformkörpers 18.

### Gewerbliche Anwendbarkeit:

Der Gegenstand der Erfindung ist als hochgenaues und hochempfindliches Meßgerät für die Messung der Zerfallszeit von Arzneiformkörpern gemäß dem DAB 10, 3. Nachtrag 1994 oder dem Europäischen Arzneibuch oder dem USP (USA) geeignet.

## Patentansprüche

1. Vorrichtung zum Bestimmen der Zerfallszeit von verpreßten Arzneiformkörpern (18), wie Tabletten und Kapseln, bestehend aus einem in ein mit einer Flüssigkeit gefülltes Gefäß (19) absenkbaren Gestell (1), mit einer Mittelsäule (7) und einem Boden (3), der eine Mehrzahl von Löchern (5, 5') enthält, die von unten mit einem Sieb (4) abgedeckt sind und in denen innerhalb des Gestells (1) Prüfröhrchen (6, 6') aufrechtstehend angeordnet sind, in welchen je eine Scheibe (8) mit durchgehenden, in Richtung der Mittelachse der Scheibe (8) verlaufenden Löchern (10,10') als Beschwerung des einzelnen Arzneiformkörpers (18) beweglich einbringbar ist, wobei an der dem Boden (3) zugewandten Unterseite der Scheibe (8) ein Dauermagnet (11) angeordnet ist, demgegenüber sich unterhalb des Netzes (4) des Bodens (3) je ein Magnetfeldsensorelement (13) befindet, dadurch gekennzeichnet,
daß das zentrale Loch (10) der Scheibe (8) zentrisch eine von der unteren Deckfläche der Scheibe (8) ausgehende Aufbohrung (28) aufweist, in der der Dauermagnet (11) sitzt, der zentrisch in Richtung der Mittelachse (27) ein Loch (25) aufweist, welches mit dem zentralen Loch (10) innerhalb der Scheibe (8) fluchtet, wobei das Magnetfeldsensorelement (13) in der Draufsicht auf den Dauermagneten (11) im Bereich des peripheren Randes der Deckfläche des Dauermagneten (11) angeordnet ist.

2. Vorrichtung nach Anspruch 1, dadurch gekennzeichnet,
dass das Magnetfeldsensorelement (13) im Bereich des größten Gradienten des magnetischen Feldes des Dauermagneten (11) angeordnet ist.

3. Vorrichtung nach Anspruch 1, dadurch gekennzeichnet,
daß der Dauermagnet (11) ein rohr- oder hülsenförmiger, zylindrischer Torus ist, dessen Längenausdehnung größer als sein Durchmesser ist.

4. Vorrichtung nach Anspruch 2 oder 3, dadurch gekennzeichnet,
daß der Dauermagnet (11) innerhalb der Scheibe (8) vollständig von Kunststoffmaterial umgeben ist.

5. Vorrichtung nach Anspruch 4, dadurch gekennzeichnet,
daß in das Loch (25) des Dauermagneten (11) eine Kunststoffhülse (12) eingesetzt ist.

6. Vorrichtung nach einem der vorherigen Ansprüche,
dadurch gekennzeichnet,
daß das Magnetfeldsensorelement (13) ein Hallgenerator oder ein magnetoresistiver Sensor oder eine Sättigungskernsonde ist.

7. Verfahren zum Bestimmen der Zerfallszeit von yerpreßten Arzneiformkörpern (18) wie Tabletten und Kapseln, mit der Vorrichtung gemäß Anspruch 1, wobei
während des Auflösungsvorgangs des Arzneiformkörpers (18) eine elektrische Mehrfachmessung mit anschließender Mittelwertbildung erfolgt.

## Claims

1. Apparatus for determining the disintegration time of compressed pharmaceutical mold bodies (18), such as tablets or capsules, comprising a frame (1) lowerable into a vessel (19) filled with a liquid, wherein the frame (1) is furnished with a center column (7) and a floor (3), which floor (3) contains a plurality of holes (5,5' ), wherein the holes (5,5') are covered from below with a sieve (4) and wherein test tubes (6,6') disposed upright are positioned in the holes (5,5' ) within the frame (1), wherein in each case a disk (8) with passing through holes (10, 10') is movably insertable as a weight of the individual pharmaceutical form body (18), wherein the holes (10, 10') are running in the direction of the center axis of the disk (8), wherein a permanent magnet (11) is disposed at the bottom side of thedisk (8) directed toward the floor (3), wherein in each case a magnetic field sensor element (13) is disposed opposite to the permanent magnet (11) below the grid (4) of the floor (3),
characterized by the center hole (10) of the disk (8) exhibits a bore hole (28) starting from the lower cover face of the disk (8), wherein the permanent magnet (11) is seated in the bore hole (28), wherein the permanent magnet (11) exhibits a hole (25) centeredly in the direction of the center axis (27), which hole (25) is aligned with a centered hole (10) within the disk (8), wherein the magnetic field sensor element (13) is disposed in the region of the peripheral edge of the cover face of the permanent magnet (11) when seen in a top view onto the permanent magnet (11).

2. Apparatus according to claim 1, wherin the magnetic field sensor element (13) is situated in the region of the largest gradient of the magnetic field of the permanent magnet (11).

3. Apparatus according to claim 1, wherein the permanent magnet (11) is formed as a tubular shaped or sleeve shaped cylindrical torus, wherein the longitudinal extension of the torus is larger than the diameter of the torus.

4. Apparatus according to claim 2 or 3, wherein the permanent magnet (11) is surrounded completely within the disk (8) by plastic material.

5. Apparatus according to claim 4 wherein a plastic sleeve (12) is inserted into the hole (25) of the permanent magnet (11).

6. Apparatus according to one of the preceding claims, wherein the magnetic field sensor element (13) is a Hall generator, or a magneto-resistive sensor or a saturable core probe.

7. Method for determining the disintegration time of compressed pharmaceutical mold bodies (18) such as tablets and capsules, with the aid of the apparatus according to claim 1, wherein an electrical multiple measurement with successive average value formation is performed during the dissolution process of the medical mold body (18).

## Revendications

1. Dispositif pour déterminer le temps de désagrégation de corps moulés de médicaments compacts (18), tels que des comprimés et des capsules, comprenant un support (1) que l'on peut faire descendre dans un récipient (19) rempli d'un liquide et présentant une colonne centrale (7) et un fond (3) comportant une pluralité de trous (5, 5') recouverts par le bas par un tamis (4) et dans lesquels, à l'intérieur du support (1) sont placées toutes droites des éprouvettes (6, 6'), dans chacune desquelles un disque (8) comportant des trous qui le traversent (10, 10') s'étendant dans le sens de l'axe médian du disque (8) peut être introduit comme poids de charge des corps moulés de médicaments (18) individuels, et un aimant permanent (11) étant disposé sur la face inférieure du disque (8) dirigée vers le fond (3), en face duquel, en-dessous du tamis (4) du fond (3), se trouve un élément capteur de champ magnétique (13), caractérisé en ce que
le trou central (10) du disque (8) est élargi par un alésage (28) central partant de la face inférieure du disque (8) et hébergeant l'aimant permanent (11) qui, dans le sens de l'axe médian (27), présente un trou central (25) lequel est aligné sur le trou central (10) du disque (8), l'élément capteur de champ magnétique (13) étant disposé, vu d'en haut sur l'aimant permanent (11), dans la zone du bord périphérique de la surface de recouvrement de l'aimant permanent (11).

2. Dispositif selon la revendication 1, caractérisé en ce que l'élément capteur de champ magnétique (13) est disposé dans la zone du gradient le plus important du champ magnétique de l'aimant permanent (11).

3. Dispositif selon la revendication 1, caractérisé en ce que l'aimant permanent (11) est un tore ayant la forme d'un tube, d'une douille ou d'un cylindre, dont la longueur est plus grande que son diamètre.

4. Dispositif selon la revendication 2 ou 3, caractérisé en ce que l'aimant permanent (11) est complètement entouré de matière plastique à l'intérieur du disque (8).

5. Dispositif selon la revendication 4, caractérisé en ce qu'une douille en matière plastique (12) est insérée dans le trou (25) de l'aimant permanent (11).

6. Dispositif selon l'une quelconque des revendication précédentes, caractérisé en ce que l'élément capteur de champ magnétique (13) est un générateur à effet de Hall ou un capteur magnéto-résistif ou une sonde à saturation.

7. Procédé pour déterminer, à l'aide du dispositif selon la revendication 1, le temps de désagrégration de corps moulés de médicaments compacts (18) tels que des comprimés et des capsules, dans lequel de multiples mesures électriques, suivies de la détermination de la valeur moyenne, sont effectuées lors du processus de désagrégation du corps moulé de médicament compact (18).
